# EUROPEAN PATENT APPLICATION

(11) **EP 4 451 219 A1**
(43) Date of publication of application: **23.10.2024**
(21) Application number: 24169864.6
(22) Date of filing: 12.04.2024
(51) Int. Cl.: G06T 11/00

(54) **METHOD AND APPARATUS FOR TRAINING GENERATIVE MODEL FOR MEDICAL IMAGE ASSOCIATED WITH PLURALITY OF BODY PARTS**

(30) Priority: 19.04.2023 KR 20230051496
(71) Applicant: GenGenAI Inc., Seoul 06038 (KR)
(72) Inventor: CHO, Hojin, 13561 Seongnam-si (KR); KIM, Sangil, 13561 Seongnam-si (KR); HEO, Hoyeong, 13561 Seongnam-si (KR)
(74) Representative: Studio Torta S.p.A.

(57) **Abstract**

Provided is a method for training a generative model for medical images associated with a plurality of body parts, which is performed by one or more processors and includes receiving medical image data, wherein the medical image data is associated with a plurality of body parts and configured for training a generative model for medical images, acquiring label data associated with the medical image data, wherein the label data comprises a score associated with an anatomical location of at least one body part of the plurality of body parts, and training, based on the received medical image data and the acquired label data, the generative model for medical images.

## Description

### TECHNICAL FIELD

The present disclosure relates to method and apparatus for training a generative model for medical images associated with a plurality of body parts in a 3D space, and specifically, to method and system for training a generative model for medical images based on training medical image data and label data associated with the training medical image data.

### BACKGROUND

In recent years, advances in deep learning technologies and improvements in computing power have led to improved performance and increased diversity of artificial intelligence (AI) generative models. As a result, the generative models receive increasing attention in the artificial intelligence research and applications. The generative models are expected to be utilized in various fields such as creation of new contents, prediction, decision-making, and simulation, and many studies and developments are being conducted to utilize them in various fields.

Meanwhile, the generative models are also the subject of interest in the medical field, and attempts are being made to utilize them. In healthcare, generative models learn from large amounts of medical data, embedding medical knowledge into artificial intelligence-based models and generating new data to be utilized to aid in medical research, diagnosis, and treatment, and in particular, generative models that generate medical images generate images such as CT, MRI, and X-ray of patients to be used for medical image analysis, diagnostic assistance, and surgical simulation.

However, related training methods for the generative model for medical images have problems of requiring participation of anatomy experts for selecting the body parts to be learned, classifying the training data, labeling the training data, and generating and training a plurality of models for each body part to be imaged.

### SUMMARY

In order to solve one or more problems (e.g., the problems described above and/or other problems not explicitly described herein), the present disclosure provides method and apparatus (system) for training a generative model for medical images associated with a plurality of body parts.

The present disclosure may be implemented in a variety of ways, including a method, an apparatus (system), or a non-transitory computer-readable recording medium storing instructions.

A method for training a generative model for medical images associated with a plurality of body parts may be performed by one or more processors and may include receiving medical image data, in which the medical image data is associated with a plurality of body parts and configured for training a generative model for medical images, acquiring label data associated with the medical image data, in which the label data comprises a score associated with an anatomical location of at least one body part of the plurality of body parts and training, based on the received medical image data and the acquired label data, the generative model for medical images.

The medical image data may include a plurality of two-dimensional (2D) computed tomography (CT) slice real images, and the generative model for medical images may be trained to receive at least one of a number or a text representing a specific anatomical location of a body part and generate at least one 2D CT slice synthetic image associated with the specific anatomical location.

The method may further include generating, based on the generative model for medical images, the at least one 2D CT slice synthetic image associated with the specific anatomical location and outputting the generated at least one 2D CT slice synthetic image associated with the specific anatomical location.

The score may include a body part regression (BPR) score quantifying an anatomical location of a body part associated with each of the medical image data.

The label data may be generated by inputting the medical image data to a machine learning BPR model that is trained to estimate the BPR score.

The medical image data may include a plurality of two-dimensional (2D) computed tomography (CT) slice real images and relative location information for each of the plurality of 2D CT slice real images, and based on a determination that the machine learning BPR model fails to estimate a BPR score of a specific 2D CT slice real image of the plurality of 2D CT slice real images, a 2D CT slice real image of an adjacent location may be identified using the relative location information, and the BPR score of the specific 2D CT slice real image may be estimated based on the BPR score of the 2D CT slice real image of the adjacent location.

The method may further include normalizing the label data to a specific range.

The method may further include sampling, based on a specific body part regression (BPR) score or a specific range of BPR scores, the label data and the medical image data, and the training the generative model for medical images may include training, based on the sampled label data and the sampled medical image data, the generative model for medical images.

A computer program stored in a computer-readable recording medium may be provided to execute the method on a computer.

A system may be provided, which may include a memory and one or more processors connected to the memory and configured to execute one or more computer-readable programs included in the memory, in which the one or more programs may include instructions for receiving a user input indicating a specific anatomical location of a body part of a plurality of body parts, inputting the user input into a generative model for medical images to generate a medical image associated with the specific anatomical location, and generating, based on the generative model for medical images, the medical image associated with the specific anatomical location, the generative model for medical images may be a model that is trained based on medical image data and label data associated with the medical image data, and the label data may include a score associated with an anatomical location of at least one body part of the plurality of body parts.

The label data and the medical image data may be sampled based on a specific body part regression (BPR) score or a specific range of BPR scores, and the generative model for medical image may be trained based on the sampled label data and the sampled medical image data.

The user input may include at least one of a body part regression (BPR) score quantifying the specific anatomical location or a text indicating the specific anatomical location.

The user input may include a single BPR score, and the generated medical image may be a two-dimensional (2D) medical image corresponding to the specific anatomical location.

The user input may be a specific range of BPR scores, and the generated medical image may be a 3D medical image corresponding to the specific anatomical location.

The user input may be a specific range of BPR scores, and the generated medical image may be a three-dimensional (3D) medical image corresponding to the specific anatomical location.

The generative model for medical images may be a single generative model that is trained to generate medical images associated with a plurality of body parts.

According to various examples of the present disclosure, by training the generative model for medical images based on the training medical image data associated with a plurality of body parts and the label data, it is possible to train the generative model for medical images to generate medical images associated with the plurality of body parts in a single model.

According to various examples of the present disclosure, by automating labeling with the machine learning model that is trained to estimate the label data, it is possible to reduce the time cost and monetary cost required for classifying training data.

According to various examples of the present disclosure, by sampling the training medical image data and selecting only partial or representative data for model training, it is possible to perform model training efficiently. Further, by using sampled training medical image data for model training based on user input (e.g., specific BPR score, specific range of BPR scores), it is possible to train the generative model to generate medical images targeting a specific body part or a specific range of body parts.

According to various examples of the present disclosure, by using a single generative model for medical images to generate 2D or 3D medical images associated with specific anatomical locations from a variety of input data (e.g., a single BPR score, a specific range of BPR scores, text, etc.), it is possible to generate and provide medical images that are customized to the needs of a consumer requiring medical images.

The effects of the present disclosure are not limited to the effects described above, and other effects not described herein can be clearly understood by those of ordinary skill in the art (referred to as "ordinary technician") from the description of the claims.

### BRIEF DESCRIPTION OF THE DRAWINGS

The above and other objects, features and advantages of the present disclosure will be described with reference to the accompanying drawings described below, where similar reference numerals indicate similar elements, but not limited thereto, in which:
FIG. 1 illustrates an example of a method for training a generative model for medical images;
FIG. 2 is a schematic diagram illustrating a configuration in which an information processing system is communicatively coupled to a plurality of user terminals to provide a trained generative model for medical images and/or generated medical image data;
FIG. 3 is a block diagram of an internal configuration of the user terminal and the information processing system;
FIG. 4 is a block diagram illustrating an internal configuration of a processor of the information processing system;
FIG. 5 is a diagram illustrating an example of a label data acquisition unit acquiring label data;
FIG. 6 is a diagram illustrating an example of a data sampling unit sampling training medical image data;
FIG. 7 is a diagram illustrating an example of generating a medical synthetic image from a single BPR score;
FIG. 8 is a diagram illustrating examples of the generated medical synthetic images;
FIG. 9 is a diagram illustrating an example of generating a medical synthetic image from a specific range of BPR scores;
FIG. 10 is a diagram illustrating an example of generating a medical synthetic image from text data;
FIG. 11 a diagram illustrating an example of a machine learning model; and
FIG. 12 is a flowchart illustrating an example of a method for training a generative model for medical images.

### DETAILED DESCRIPTION

Hereinafter, example details for the practice of the present disclosure will be described in detail with reference to the accompanying drawings. However, in the following description, detailed descriptions of well-known functions or configurations will be omitted if it may make the subject matter of the present disclosure rather unclear.

In the accompanying drawings, the same or corresponding components are assigned the same reference numerals. In addition, in the following description of various examples, duplicate descriptions of the same or corresponding components may be omitted. However, even if descriptions of components are omitted, it is not intended that such components are not included in any example.

Advantages and features of the disclosed examples and methods of accomplishing the same will be apparent by referring to examples described below in connection with the accompanying drawings. However, the present disclosure is not limited to the examples disclosed below, and may be implemented in various forms different from each other, and the examples are merely provided to make the present disclosure complete, and to fully disclose the scope of the disclosure to those skilled in the art to which the present disclosure pertains.

The terms used herein will be briefly described prior to describing the disclosed example(s) in detail. The terms used herein have been selected as general terms which are widely used at present in consideration of the functions of the present disclosure, and this may be altered according to the intent of an operator skilled in the art, related practice, or introduction of new technology. In addition, in specific cases, certain terms may be arbitrarily selected by the applicant, and the meaning of the terms will be described in detail in a corresponding description of the example(s). Therefore, the terms used in the present disclosure should be defined based on the meaning of the terms and the overall content of the present disclosure rather than a simple name of each of the terms.

The singular forms "a," "an," and "the" as used herein are intended to include the plural forms as well, unless the context clearly indicates the singular forms. Further, the plural forms are intended to include the singular forms as well, unless the context clearly indicates the plural forms. Further, throughout the description, when a portion is stated as "comprising (including)" a component, it is intended as meaning that the portion may additionally comprise (or include or have) another component, rather than excluding the same, unless specified to the contrary.

Further, the term "module" or "unit" used herein refers to a software or hardware component, and "module" or "unit" performs certain roles. However, the meaning of the "module" or "unit" is not limited to software or hardware. The "module" or "unit" may be configured to be in an addressable storage medium or configured to play one or more processors. Accordingly, as an example, the "module" or "unit" may include components such as software components, object-oriented software components, class components, and task components, and at least one of processes, functions, attributes, procedures, subroutines, program code segments, drivers, firmware, micro-codes, circuits, data, database, data structures, tables, arrays, and variables. Furthermore, functions provided in the components and the "modules" or "units" may be combined into a smaller number of components and "modules" or "units", or further divided into additional components and "modules" or "units."

The "module" or "unit" may be implemented as a processor and a memory. The "processor" should be interpreted broadly to encompass a general-purpose processor, a Central Processing Unit (CPU), a microprocessor, a Digital Signal Processor (DSP), a controller, a microcontroller, a state machine, and so forth. Under some circumstances, the "processor" may refer to an application-specific integrated circuit (ASIC), a programmable logic device (PLD), a field-programmable gate array (FPGA), and so on. The "processor" may refer to a combination for processing devices, e.g., a combination of a DSP and a microprocessor, a combination of a plurality of microprocessors, a combination of one or more microprocessors in conjunction with a DSP core, or any other combination of such configurations. In addition, the "memory" should be interpreted broadly to encompass any electronic component that is capable of storing electronic information. The "memory" may refer to various types of processor-readable media such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or optical data storage, registers, and so on. The memory is said to be in electronic communication with a processor if the processor can read information from and/or write information to the memory. The memory integrated with the processor is in electronic communication with the processor.

In the present disclosure, a "system" may refer to at least one of a server apparatus and a cloud apparatus, but is not limited thereto. For example, the system may include one or more server apparatus. In another example, the system may include one or more cloud apparatus. In still another example, the system may include both the server apparatus and the cloud apparatus operated in conjunction with each other.

In the present disclosure, a "machine learning model" may include any model that is used for inferring an answer to a given input. The machine learning model may include an artificial neural network model including an input layer, a plurality of hidden layers, and an output layer. Each layer may include a plurality of nodes.

In the present disclosure, "medical image data" may refer to 2D image data or 3D image data utilized in the field of medicine. For example, the medical image data may include 3D scan data (e.g., CT images, MRI images, etc.) of human body or organs.

In the present disclosure, "Body Part Regression (BPR)" may refer to a technique that utilizes artificial intelligence and computer vision techniques to identify a human body part in an image or video and estimate the location or shape of the part. Further, a "BPR score" may refer to a score that quantifies the anatomical location of a corresponding body part by utilizing the BPR technology.

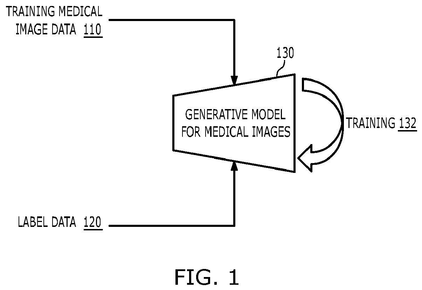

FIG. 1 illustrates an example of a method for training a generative model 130 for medical images. Training medical image data 110 may be received to train the generative model 130 for medical images. As for the training medical image data 110, various training data may be received and acquired according to the purpose of training the generative model for medical images. For example, if the model is trained to generate medical images for the entire body, the training medical image data 110 may be medical image data for various body parts. As another example, for training a medical image model specialized in generating medical images of a specific body part, the training medical image data 110 may be medical image data for a specific range of body parts or medical image data for a single body part.

The training medical image data 110 may be various forms of 2D or 3D image data utilized in the medical field, and may be real data or data generated by data augmentation. Further, the training medical image data 110 may include relative location information for each of the medical images. For example, the training medical image data 110 may be a plurality of 2D CT slice real images, and may include relative location information for each of the plurality of 2D CT slice real images (e.g., CT slice image 3 is a CT slice image for a location between CT slice image 2 and CT slice image 4).

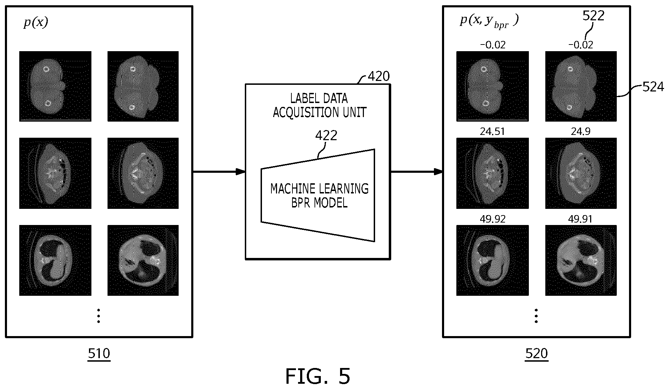

Label data 120 associated with the training medical image data may be acquired to train the generative model 130 for medical images. The label data 120 may refer to data obtained by labeling details associated with the training medical image data 110 to train the generative model 130 for medical images. For example, the label data 120 may be the BPR score quantifying the anatomical location of the body part associated with each of the training medical image data 110, and may be generated by inputting the training medical image data 110 into a machine learning BPR model that is trained to estimate the BPR score. This will be described in detail elsewhere with reference to FIG. 5. As another example, the label data 120 may be BPR scores labeled by an expert in medical imaging (e.g., an anatomy expert, medical practitioner, etc.).

The label data 120 may be estimated based on the relative location information for each of the training medical images included in the training medical image data 110. For example, if the machine learning BPR model fails to estimate a BPR score for a specific 2D CT slice image of a plurality of 2D CT slice images, at least one 2D slice image at an adjacent location may be identified using the relative location information, and the BPR score for the specific 2D CT slice image may be estimated based on the BPR scores of the 2D CT slice images at the identified adjacent location.

The generative model 130 for medical images may be trained (e.g., machine learned) (132) based on the training medical image data 110 and the label data 120. For example, the generative model 130 for medical images may be trained using the training data that may include pairs of the training medical image data 110 and the corresponding label data 120. In this case, the generative model 130 for medical images may be a model that mimics the distribution of the training medical image data 110 and the label data 120 to generate medical image data close to the actual distribution of the data, and may be a model that receives and learns the label data 120 together so as to generate data corresponding to a specific label. For example, the generative model 130 for medical images may be a single generative model that is trained to generate medical images associated with a plurality of body parts.

The generative model 130 for medical images may be a model that receives user input indicative of a specific anatomical location of a body part and generates medical images associated with the specific anatomical location. For example, the generative model 130 for medical images may be a generative model that is trained to receive at least one of a number or a text representing a specific anatomical location of a body part and generate at least one 2D CT slice synthetic image associated with the corresponding specific anatomical location.

As in the configuration described above, by training the generative model for medical images based on the training medical image data associated with a plurality of body parts and the label data, it is possible to train the generative model for medical images to generate medical images associated with the plurality of body parts in a single model.

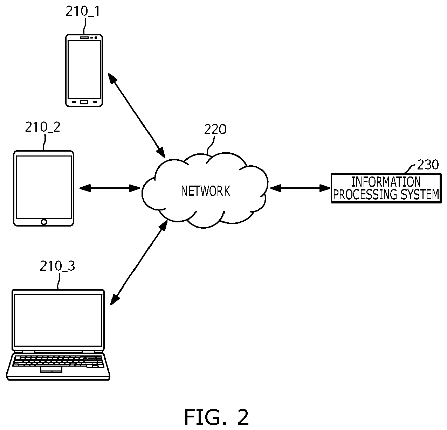

FIG. 2 schematically illustrates a configuration in which an information processing system 230 is communicatively connected to a plurality of user terminals 210_1, 210_2, and 210_3 to provide a trained generative model for medical images, generated medical image data, etc. As illustrated, the plurality of user terminals 210_1, 210_2, and 210_3 may be connected through a network 220 to the information processing system 230 which can provide a generative model for medical images, medical image data, etc. The plurality of user terminals 210_1, 210_2, and 210_3 may include user terminals that receive the generative model for medical images, the medical image data, etc.

The information processing system 230 may include one or more server devices and/or databases or one or more distributed computing devices and/or distributed databases based on cloud computing services that can store, provide and execute computer-executable programs (e.g., downloadable applications) and data associated with the provision of generative model for medical images, medical image data, etc.

The generative model for medical images and/or the medical image data provided by the information processing system 230 may be provided to the user through an application web browser or web browser extension program related to the generative model for medical image data and/or the medical image data, etc. of each of the plurality of user terminals 210_1, 210_2, and 210_3. For example, the information processing system 230 may provide corresponding information or perform a corresponding process according to a request to provide the generative model for medical images, a request to generate medical image data, etc. received from the user terminals 210_1, 210_2, and 210_3 through an application related to generative model for medical images, an application related to medical image data, etc.

The plurality of user terminals 210_1, 210_2, and 210_3 may communicate with the information processing system 230 through the network 220. The network 220 may be configured to enable communication between the plurality of user terminals 210_1, 210_2, and 210_3 and the information processing system 230. The network 220 may be configured as a wired network such as Ethernet, a wired home network (Power Line Communication), a telephone line communication device and RS-serial communication, a wireless network such as a mobile communication network, a wireless LAN (WLAN), Wi-Fi, Bluetooth, and ZigBee, or a combination thereof, depending on the installation environment. The method of communication may include a communication method using a communication network (e.g., mobile communication network, wired Internet, wireless Internet, broadcasting network, satellite network, etc.) that may be included in the network 220 as well as short-range wireless communication between the user terminals 210_1, 210_2, and 210_3, but aspects are not limited thereto.

In FIG. 2, a mobile phone terminal 210_1, a tablet terminal 210_2, and a PC terminal 210_3 are illustrated as the examples of the user terminals, but aspects are not limited thereto, and the user terminals 210_1, 210_2, and 210_3 may be any computing device that is capable of wired and/or wireless communication and capable of installing and executing a generative model for medical images, an application or web browser related to medical image data, etc. For example, the user terminal may include an AI speaker, a smart phone, a mobile phone, a navigation, a computer, a notebook, a digital broadcasting terminal, a personal digital assistant (PDA), a portable multimedia player (PMP), a tablet PC, a game console, a wearable device, an internet of things (IoT) device, a virtual reality (VR) device, an augmented reality (AR) device, a set-top box, etc. In addition, FIG. 2 illustrates that three user terminals 210_1, 210_2, and 210_3 are in communication with the information processing system 230 through the network 220, but aspects are not limited thereto, and a different number of user terminals may be configured to be in communication with the information processing system 230 through the network 220.

FIG. 2 illustrates an example in which the user terminals 210_1, 210_2, and 210_3 receive a generative model for medical images, medical image data, etc. from the information processing system 230, but aspects are not limited thereto. For example, the generative model for medical image data and/or medical image data may be provided without a communication with the information processing system 230, that is, via a program, an application, etc. relating to the generative model for medical images, the medical image data, etc., installed in the user terminals 210_1, 210_2, and 210_3.

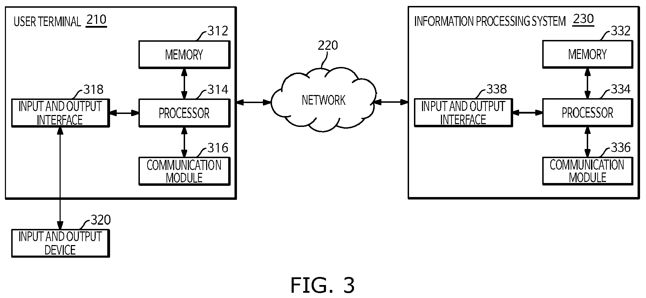

FIG. 3 is a block diagram of an internal configuration of the user terminal 210 and the information processing system 230. The user terminal 210 may refer to any computing device that is capable of executing the application, web browsers, etc., and also capable of wired/wireless communication, and may include the mobile phone terminal 210_1, the tablet terminal 210_2, and the PC terminal 210_3 of FIG. 2, for example. As illustrated, the user terminal 210 may include a memory 312, a processor 314, a communication module 316, and an input and output interface 318. Likewise, the information processing system 230 may include a memory 332, a processor 334, a communication module 336, and an input and output interface 338. As illustrated in FIG. 3, the user terminal 210 and the information processing system 230 may be configured to communicate information, data, etc. through the network 220 using respective communication modules 316 and 336. In addition, an input and output device 320 may be configured to input information, data, etc. to the user terminal 210, or output information, data, etc. generated from the user terminal 210 through the input and output interface 318.

The memories 312 and 332 may include any non-transitory computer-readable recording medium. The memories 312 and 332 may include a permanent mass storage device such as read only memory (ROM), disk drive, solid state drive (SSD), flash memory, etc. As another example, a non-destructive mass storage device such as ROM, SSD, flash memory, disk drive, etc. may be included in the user terminal 210 or the information processing system 230 as a separate permanent storage device that is distinct from the memory. In addition, an operating system and at least one program code may be stored in the memories 312 and 332.

These software components may be loaded from a computer-readable recording medium separate from the memories 312 and 332. Such a separate computer-readable recording medium may include a recording medium directly connectable to the user terminal 210 and the information processing system 230, and may include a computer-readable recording medium such as a floppy drive, a disk, a tape, a DVD/CD-ROM drive, a memory card, etc., for example. As another example, the software components may be loaded into the memories 312 and 332 through the communication modules 316 and 336 rather than the computer-readable recording medium. For example, at least one program may be loaded into the memories 312 and 332 based on a computer program installed by files provided by developers or a file distribution system that distributes an installation file of an application via the network 220.

The processors 314 and 334 may be configured to process the instructions of the computer program by performing basic arithmetic, logic, and input and output operations. The instructions may be provided to the processors 314 and 334 from the memories 312 and 332 or the communication modules 316 and 336. For example, the processors 314 and 334 may be configured to execute the received instructions according to a program code stored in a recording device such as the memories 312 and 332.

The communication modules 316 and 336 may provide a configuration or function for the user terminal 210 and the information processing system 230 to communicate with each other through the network 220, and may provide a configuration or function for the user terminal 210, the information processing system 230, etc. to communicate with another user terminal or another system (e.g., a separate cloud system, etc.). For example, a request or data (e.g., a request to provide a generative model for medical images, a request to generate medical image data, etc.) generated by the processor 314 of the user terminal 210 according to the program code stored in the recording device such as the memory 312, etc. may be transmitted to the information processing system 230 through the network 220 under the control of the communication module 316. Conversely, a control signal or command provided under the control of the processor 334 of the information processing system 230 may be received by the user terminal 210 through the communication module 316 of the user terminal 210 through the communication module 336 and the network 220.

The input and output interface 318 may be a means for interfacing with the input and output device 320. As an example, the input device may include a device such as a camera including an audio sensor and/or an image sensor, a keyboard, a microphone, a mouse, etc., and the output device may include a device such as a display, a speaker, a haptic feedback device, etc. As another example, the input and output interface 318 may be a means for interfacing with a device such as a touch screen, etc. that incorporates a configuration or function for performing inputting and outputting. For example, when the processor 314 of the user terminal 210 processes the instructions of the computer program loaded into the memory 312, a service screen, etc., which is configured with the information, data, etc. provided by the information processing system 230 or another user terminals, may be displayed on the display via the input and output interface 318. While FIG. 3 illustrates that the input and output device 320 is not included in the user terminal 210, aspects are not limited thereto, and an input and output device may be configured as one device with the user terminal 210. In addition, the input and output interface 338 of the information processing system 230 may be a means for interfacing with a device (not illustrated) for inputting or outputting, which may be connected to or included in the information processing system 230. While FIG. 3 illustrates the input and output interfaces 318 and 338 as the components configured separately from the processors 314 and 334, aspects are not limited thereto, and the input and output interfaces 318 and 338 may be configured to be included in the processors 314 and 334.

The user terminal 210 and the information processing system 230 may include more components than those illustrated in FIG. 3. Meanwhile, most of the related components may not necessarily require exact illustration. The user terminal 210 may be implemented to include at least a part of the input and output device 320 described above. In addition, the user terminal 210 may further include another component such as a transceiver, a global positioning system (GPS) module, a camera, various sensors, a database, etc. For example, the user terminal 210 that is a smartphone may generally include components included in the smartphone, and for example, may be implemented such that various components such as an acceleration sensor, a gyro sensor, a microphone module, a camera module, various physical buttons, buttons using a touch panel, input and output ports, a vibrator for vibration, etc. are further included in the user terminal 210.

While a program for the application related to the generative model for medical images, application related to the medical image data, etc. is running, the processor 314 may receive text, image, video, audio, and/or action, etc. inputted or selected through the input device such as a camera, a microphone, etc., that includes a touch screen, a keyboard, an audio sensor and/or an image sensor connected to the input and output interface 318, and store the received text, image, video, audio, and/or action, etc. in the memory 312, or provide the same to the information processing system 230 through the communication module 316 and the network 220.

The processor 314 of the user terminal 210 may be configured to manage, process and/or store the information, data, etc. received from the input and output device 320, another user terminal, the information processing system 230, a plurality of external systems, etc. The information, data, etc. processed by the processor 314 may be provided to the information processing system 230 via the communication module 316 and the network 220. The processor 314 of the user terminal 210 may transmit the information, data, etc. to the input and output device 320 via the input and output interface 318 to output the same. For example, the processor 314 may display the received information, data, etc. on a screen of the user terminal 210.

The processor 334 of the information processing system 230 may be configured to manage, process, and/or store information, data, etc. received from a plurality of user terminals 210, a plurality of external systems, etc. The information, data, etc. processed by the processor 334 may be provided to the user terminals 210 via the communication module 336 and the network 220.

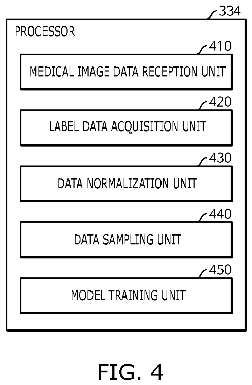

FIG. 4 is a block diagram illustrating an internal configuration of the processor 334 of the information processing system. As illustrated in FIG. 4, the processor 334 may include a medical image data reception unit 410, a label data acquisition unit 420, a data normalization unit 430, a data sampling unit 440, and a model training unit 450. The respective components of the processor 334 illustrated in FIG. 4 represent functional components that can be divided on the basis of functions, and in an actual physical environment, a plurality of components may be implemented as being integrated with each other. Alternatively, the respective components of the processor 334 may be implemented separately from each other in the actual physical environment.

The medical image data reception unit 410 may receive training medical image data to train a generative model for medical images. The medical image data reception unit 410 may receive various training data according to the purpose of training the generative model for medical images. For example, the medical image data reception unit 410 may receive training medical image data for various body parts to train a general-purpose generative model for medical images. As another example, in order to train a medical image model for a specific part, the medical image data reception unit 410 may receive training medical image data for a specific range of body parts or training medical image data for a single body part. Further, the medical image data reception unit 410 may receive various forms of 2D or 3D image data utilized in the medical field as the training medical data, and may receive actual data, data generated by data augmentation, synthetic data, etc. Additionally, the medical image data reception unit 410 may receive relative location information for each of the medical images. The medical image data reception unit 410 may receive the training medical image data from an internal storage or an external database.

The label data acquisition unit 420 may acquire, generate, or receive label data associated with the training medical image data to train the generative model for medical images. As an example of the label data, the label data acquisition unit 420 may acquire a BPR score that quantifies an anatomical location of a body part associated with each of the training medical image data. For example, the label data acquisition unit 420 may include a machine learning BPR model trained to estimate the BPR scores from the training medical image data, and acquire the BPR scores by inputting the training medical image data received by the medical image data reception unit 410 into the machine learning BPR model. This will be described in detail elsewhere with reference to FIG. 5. As another example, the label data acquisition unit 420 may acquire the BPR scores that are labeled by the expert in medical imaging (e.g., an anatomy expert, medical practitioner, etc.).

Further, the label data acquisition unit 420 may acquire label data based on the relative location information for each of the training medical images received by the medical image data reception unit 410. For example, if the machine learning BPR model of the label data acquisition unit 420 fails to estimate the BPR score of the third CT slice real image of the series of CT slice real images sets, the third slice real image may be identified based on the second CT slice real image and the fourth CT slice real image, and a 2D slice real image may be identified based on the BPR score of the second CT slice real image and the BPR score of the fourth slice real image, so that the BPR score of the third CT slice real image can be estimated by utilizing the missing value imputation technique (e.g., mean value imputation, median imputation, etc.) based on the BPR scores of the 2D CT slice real images at the identified adjacent locations.

The data normalization unit 430 may normalize the data used for training the generative model for medical images. For example, the data normalization unit 430 may scale the BPR score acquired by the label data acquisition unit 420 to a range between -10 to 110.

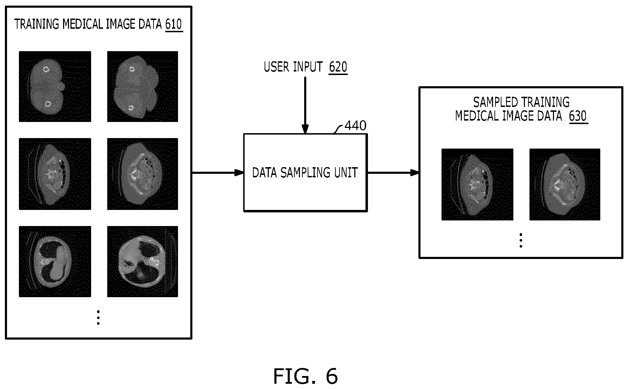

The data sampling unit 440 may sample the label data and the training medical image data based on a specific BPR score or a specific range of BPR scores. For example, the data sampling unit 440 may receive a specific BPR score or a specific range of BPR scores from a user, and sample the training medical image data based on the received BRP score. This will be described in detail below with reference to FIG. 6.

The model training unit 450 may train the generative model for medical images based on the training medical image data received by the medical image data reception unit 410 and the label data acquired by the label data acquisition unit 420. For example, the model training unit 450 may train the generative model for medical images based on pairs of training medical image data and corresponding label data. To this purpose, the model training unit 450 may train the generative model for medical images to mimic the distribution of training medical image data and label data to generate medical image data close to the actual data distribution. The generated generative model for medical images may receive the label data and generate medical synthetic image data corresponding to the corresponding labels.

In FIG. 4, the internal configuration of the processor 334 is implemented by dividing into the medical image data reception unit 410, the label data acquisition unit 420, the data normalization unit 430, the data sampling unit 440, and the model training unit 450, but aspects are not limited thereto, and some configurations may be omitted or other configurations may be added.

FIG. 5 is a diagram illustrating an example of the label data acquisition unit 420 acquiring the label data. The label data acquisition unit 420 may use a machine learning model to acquire or generate, from the training medical image data, corresponding label data or training medical image data-label data pairs. For example, training medical data 510 may be input into a machine learning BPR model 422 trained to estimate BPR scores so that a plurality of training medical image data-BPR score pairs 520 may be generated. In this case, the plurality of training medical image data-BPR score pairs 520 may include a plurality of training medical image data 524 and corresponding BPR scores 522. The generated plurality of training medical image data-BPR score pairs 520 may be used to train the generative model for medical images to mimic not only the distribution (*p*(*x*)) of the training medical images, but also the distribution (*p*(*x, y_{bpr}*)) of the training medical image data and BPR scores, thereby generating data close to the actual data distribution.

The machine learning BPR model 422 may be trained such that the label data efficiently reflects the relative location of the training medical image data. For example, the machine learning BPR model 422 may be trained to generate BPR scores in ascending or descending order based on the anatomical location of the training medical image data.

Although not illustrated in FIG. 5, if the machine learning BPR model fails to estimate the BPR score from the training medical image data, the label data acquisition unit 420 may estimate the BPR score using the relative location information of the training medical image data.

As in the configuration described above, by automating labeling with the machine learning model that is trained to estimate the label data, it is possible to reduce the time cost and monetary cost required for classifying training data.

FIG. 6 is a diagram illustrating an example of the data sampling unit 440 sampling the training medical image data. The data sampling unit 440 may generate training medical image data 630 sampled from the training medical image data 610. For example, when there is training medical image data for which the BPR score is not acquired by the machine learning model, the data sampling unit 440 may sample only the training medical image data that has the corresponding BPR score and use the same in training the generative model for medical images. As in the configuration described above, by sampling the training medical image data and selecting only partial or representative data for model training, it is possible to perform model training efficiently.

The data sampling unit 440 may receive a user input 620 that is a reference for sampling. For example, the user input 620 may be a text, a specific BPR score or a specific range of BPR scores that is the basis for the sampling. The text may be a text representing a specific body part (e.g., the heart, etc.), and the text may be converted into a specific BPR score or a specific range of BPR scores using a mapping table, etc. The data sampling unit 440 may sample the training medical image data based on the received user input 620. As in the configuration described above, by using the sampled training medical image data for model training based on the user input (e.g., text, specific BPR score, specific range of BPR scores), it is possible to train the generative model to generate medical images targeting a specific body part or a specific range of body parts.

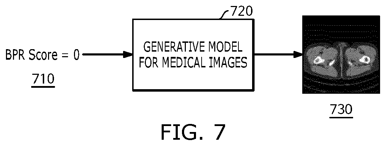

FIG. 7 is a diagram illustrating an example of generating a medical synthetic image 730 from a single BPR score 710. The single BPR score 710 quantifying a specific anatomical location of a body part may be received and the received single BPR score 710 may be input to a generative model 720 for medical images so that the medical synthetic image 730 associated with the specific anatomical location can be generated. For example, the generative model 720 for medical images may receive a BPR score of 0 and may generate a 2D medical synthetic image (e.g., a CT slice image) of a pelvic portion corresponding to the BPR score of 0.

Although FIG. 7 illustrates that a single 2D medical synthetic image corresponding to a single BPR score is generated, aspects are not limited thereto, and a plurality of 2D medical synthetic images or 3D medical synthetic images may be generated. In addition, rather than a single BPR score, the generative model 720 for medical images may receive a specific range of BPR scores or texts and generate 2D or 3D medical synthetic image.

The generative model 720 for medical images may be a model trained based on the training medical images and label data associated with the training medical image data, and it may be a model trained by a method for training the generative model for medical images associated a plurality of body parts described above.

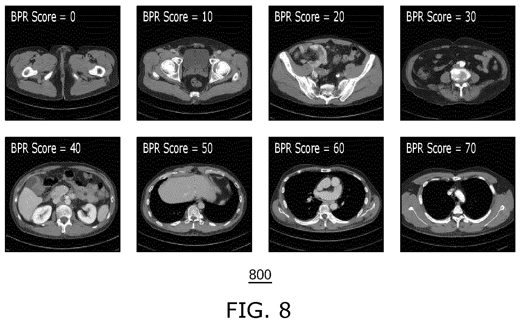

FIG. 8 is a diagram illustrating examples of the generated medical synthetic images 800. The generated medical synthetic images 800 may include a medical image generated from a BPR score by the machine learning model (generative model). For example, as illustrated in FIG. 8, BPR scores 0 to 70 may be input into the machine learning model, respectively, and CT slice synthetic images (e.g., CT slice synthetic images of the pelvis) associated with the anatomical locations corresponding to the input BPR scores may be generated.

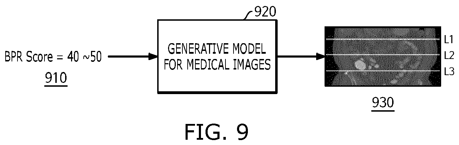

FIG. 9 is a diagram illustrating an example of generating a medical synthetic image 930 from a specific range of BPR scores 910. The specific range of BPR scores 910 quantifying a specific anatomical location of a body part may be received and the received specific range of BPR scores 910 may be input to a generative model 920 for medical images so that the medical synthetic image 930 associated with the specific anatomical location can be generated. For example, the generative model 920 for medical images may receive BPR scores of 40 to 50, and may generate a sequence of 3D medical synthetic images (e.g., 3D scan data) or 2D medical synthetic images of the mid-abdominal portion corresponding to the BPR score of 40 to 50. The 3D medical synthetic image is illustrated as an example of the medical synthetic image 930 generated in FIG. 9, but aspects are not limited thereto, and a 2D medical synthetic image (e.g., a specific range of CT scan images) may be included.

The generative model 920 for medical images may be a model trained based on the training medical images and label data associated with the training medical image data, and it may be a model trained by a method for training the generative model for medical images associated a plurality of body parts described above.

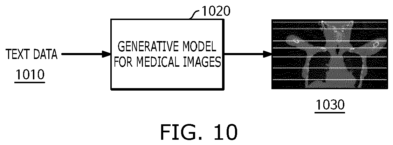

FIG. 10 is a diagram illustrating an example of generating a medical synthetic image 1030 from text data 1010. The text data 1010 indicating a specific anatomical location of a body part may be received, and the received text data 1010 may be input to a generative model 1020 for medical images so that the medical synthetic image 1030 associated with the specific anatomical location can be generated. The generated medical synthetic image 1030 may be 2D medical synthetic image (e.g., CT slice synthetic image) or 3D medical synthetic image (e.g., 3D scan data) associated with the specific anatomical location.

The generative model 1020 for medical images may be a model trained based on the training medical images and label data associated with the training medical image data, and it may be a model trained by a method for training the generative model for medical images associated a plurality of body parts described above. In addition, although not illustrated in FIG. 10, the generative model 1020 for medical images may convert the received and input text data 1010 into a specific range of BPR scores and use the result to generate the medical synthetic image 1030. For example, a mapping table may be used to convert the text data 1010 into the BPR scores.

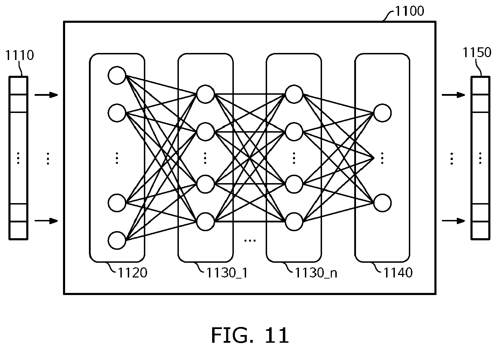

FIG. 11 a diagram illustrating an example of the machine learning model. An artificial neural network model 1100 may be used as the machine learning model. In machine learning technology and cognitive science, the artificial neural network model 1100 as an example of the machine learning model refers to a statistical learning algorithm implemented based on a structure of a biological neural network, or to a structure that executes such algorithm.

The artificial neural network model 1100 may represent a machine learning model that acquires a problem solving ability by repeatedly adjusting the weights of synapses by the nodes that are artificial neurons forming the network through synaptic combinations as in the biological neural networks, thus training to reduce errors between a target output corresponding to a specific input and a deduced output. For example, the artificial neural network model 1100 may include any probability model, neural network model, and the like, that is used in artificial intelligence learning methods such as machine learning and deep learning.

A first machine learning model for generating label data from the training medical image data may be generated in the form of the artificial neural network model 1100. For example, the artificial neural network model 1100 may receive training medical image data and generate a BPR score or a plurality of training medical image data-BPR score pairs corresponding to the training medical image. In another aspect, a second machine learning model for generating a medical image associated with a specific anatomical location of a body part from a user input (e.g., single BPR score, a specific range of BPR scores, text data, etc.) indicating the specific anatomical location may be generated in the form of the artificial neural network model 1100. For example, the artificial neural network model 1100 may receive a single BPR score and generate a 2D medical image corresponding to a specific anatomical location.

The artificial neural network model 1100 is implemented as a multilayer perceptron (MLP) formed of multiple nodes and connections between them. The artificial neural network model 1100 may be implemented using one of various artificial neural network model structures including the MLP. As illustrated in FIG. 11, the artificial neural network model 1100 includes an input layer 1120 to receive an input signal or data 1110 from the outside, an output layer 1140 to output an output signal or data 1150 corresponding to the input data, and (n) number of hidden layers 1130_1 to 1130_n (where n is a positive integer) positioned between the input layer 1120 and the output layer 1140 to receive a signal from the input layer 1120, extract the features, and transmit the features to the output layer 1140. In an example, the output layer 1140 receives signals from the hidden layers 1130_1 to 1130_n and outputs the signals to the outside.

The method of training the artificial neural network model 1100 includes the supervised learning that trains to optimize for solving a problem with inputs of teacher signals (correct answers), and the unsupervised learning that does not require a teacher signal. A computing device (e.g., information processing system, etc.) may train the artificial neural network model 1100 to generate corresponding label data from the training medical image data, using the training data including the training medical image data and the label data for the first machine learning model. In addition, the computing device may train the artificial neural network model 1100 to generate medical image data from a user input using the training data including the training medical image data and the label data for the second machine learning model.

The input variable of the artificial neural network model 1100 in the first machine learning model may include the training medical image data. As described above, if the input variable is input through the input layer 1120, the output variable output from the output layer 1140 of the artificial neural network model 1100 may be the label data (e.g., BPR score, etc.).

The input variable of the artificial neural network model 1100 in the second machine learning model may include the user input (e.g., single BPR score, a specific range of BPR scores, text data, etc.) indicating a specific anatomical location of a body part. In addition, it may be a medical image associated with a specific anatomical location output from the output layer 1140 of the artificial neural network model 1100.

As described above, the input layer 1120 and the output layer 1140 of the artificial neural network model 1100 are respectively matched with a plurality of output variables corresponding to a plurality of input variables, and as the synaptic values between nodes included in the input layer 1120, and the hidden layers 1130_1 to 1130_n, and the output layer 1140 are adjusted, training can be processed to extract a correct output corresponding to a specific input. Through this training process, the features hidden in the input variables of the artificial neural network model 1100 can be confirmed, and the synaptic values (or weights) between the nodes of the artificial neural network model 1100 can be adjusted so that there can be a reduced error between the target output and the output variable calculated based on the input variable.

The computing device may receive the training medical image data and train the first machine learning model so as to minimize a loss between label data that is ground truth and label data that is output from the first machine learning model. The label data that is the ground truth may be acquired by an expert in medical imaging (e.g., anatomy expert, medical practitioner, etc.).

In another aspect, the computing device may receive a user input indicating a specific anatomical location and train the second machine learning model so that the second machine learning model generates a medical image close to the actual medical image by way of minimizing a loss between the medical image associated with the specific anatomical location that is ground truth and the medical image output from the second machine learning model.

Using the trained artificial neural network model 1100, the label data corresponding to the training medical image data or the training medical image data-label data pairs may be generated from the first machine learning model. In addition, a medical image associated with the specific anatomical location may be generated from the second machine learning model.

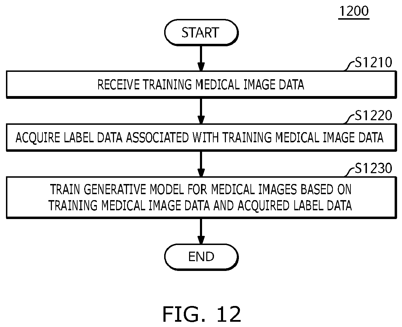

FIG. 12 is a flowchart illustrating an example of a method 1200 for training a generative model for medical images. The method 1200 may be performed by one or more processors of the user terminal or the information processing system. Alternatively, the operations of the method 1200 may be performed separately by one or more processors of the user terminal and one or more processors of the information processing system. The method 1200 may be initiated by receiving training medical image data, at S 1210.

The processor may acquire label data associated with the training medical image data, at S 1220. The label data may include a BPR score quantifying an anatomical location of a body part associated with each of the training medical image data. Moreover, the label data may be generated by inputting the training medical image data to a machine learning BPR model that is trained to estimate the BPR score.

The training medical image data may include a plurality of 2D CT slice real images and relative location information for each of the plurality of 2D CT slice real images, and if the machine learning BPR model fails to estimate the BPR score of a specific 2D CT slice real image of the plurality of 2D CT slice real images, a 2D CT slice real image of an adjacent location may be identified using the relative location information, and a BPR score of the specific 2D CT slice real image may be estimated based on the BPR score of the identified 2D CT slice real image of the adjacent location.

Finally, the processor may train the generative model for medical images based on the training medical image data and the acquired label data, at S 1230. The training medical image data may include a plurality of 2D CT slice real images, and the generative model for medical images may be trained to receive at least one of a number or a text representing a specific anatomical location of a body part and generate at least one 2D CT slice synthetic image associated with the specific anatomical location.

The processor may normalize the label data to a specific range. In addition, the processor may sample the label data and the training medical image data based on a specific BPR score or a specific range of BPR scores, and the processor may train the generative model for medical images based on the sampled label data and the sampled training medical image data.

The flowchart illustrated in FIG. 12 and the above description are merely examples, and may be implemented differently in some other examples. For example, in some examples, the order of respective steps may be changed, some steps may be repeatedly performed, some steps may be omitted, or some steps may be added.

Although not illustrated, the processor may generate a medical image (medical synthetic image) by using the trained medical image model. Specifically, the processor may receive user input indicating a specific anatomical location of a body part, and input the user input into a generative model for medical images to generate a medical image associated with the specific anatomical location. The generative model for medical images may be a model that is trained based on the training medical image data and the label data associated with the training medical image data.

The user input may include at least one of a body part regression (BPR) score quantifying a specific anatomical location or a text indicating the anatomical location. If the user input is a single BPR score, the generated medical image may be a 2D medical image (2D medical synthetic image) corresponding to the specific anatomical location. Additionally or alternatively, if the user input is a specific range of BPR scores, the generated medical image may be a 3D medical image corresponding to the specific anatomical location.

The generative model for medical images may be a single generative model that is trained to generate a medical image associated with a plurality of body parts.

The method described above may be provided as a computer program stored in a computer-readable recording medium for launch on a computer. The medium may be a type of medium that continuously stores a program executable by a computer, or temporarily stores the program for execution or download. In addition, the medium may be a variety of recording means or storage means having a single piece of hardware or a combination of several pieces of hardware, and is not limited to a medium that is directly connected to any computer system, and accordingly, may be present on a network in a distributed manner. An example of the medium includes a medium configured to store program instructions, including a magnetic medium such as a hard disk, a floppy disk, and a magnetic tape, an optical medium such as a CD-ROM and a DVD, a magnetic-optical medium such as a floptical disk, and a ROM, a RAM, a flash memory, etc. In addition, other examples of the medium may include an app store that distributes applications, a site that supplies or distributes various software, and a recording medium or a storage medium managed by a server.

The methods, operations, or techniques of the present disclosure may be implemented by various means. For example, these techniques may be implemented in hardware, firmware, software, or a combination thereof. Those skilled in the art will further appreciate that various illustrative logical blocks, modules, circuits, and algorithm steps described in connection with the disclosure herein may be implemented in electronic hardware, computer software, or combinations of both. To clearly illustrate this interchangeability of hardware and software, various illustrative components, blocks, modules, circuits, and steps have been described above generally in terms of their functionality. Whether such a function is implemented as hardware or software varies depending on design requirements imposed on the particular application and the overall system. Those skilled in the art may implement the described functions in varying ways for each particular application, but such implementation should not be interpreted as causing a departure from the scope of the present disclosure.

In a hardware implementation, processing units used to perform the techniques may be implemented in one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described in the present disclosure, computer, or a combination thereof.

Accordingly, various example logic blocks, modules, and circuits described in connection with the present disclosure may be implemented or performed with general purpose processors, DSPs, ASICs, FPGAs or other programmable logic devices, discrete gate or transistor logic, discrete hardware components, or any combination of those designed to perform the functions described herein. The general purpose processor may be a microprocessor, but in the alternative, the processor may be any related processor, controller, microcontroller, or state machine. The processor may also be implemented as a combination of computing devices, for example, a DSP and microprocessor, a plurality of microprocessors, one or more microprocessors associated with a DSP core, or any other combination of the configurations.

In the implementation using firmware and/or software, the techniques may be implemented with instructions stored on a computer-readable medium, such as random access memory (RAM), read-only memory (ROM), non-volatile random access memory (NVRAM), programmable read-only memory (PROM), erasable programmable read-only memory (EPROM), electrically erasable PROM (EEPROM), flash memory, compact disc (CD), magnetic or optical data storage devices, etc. The instructions may be executable by one or more processors, and may cause the processor(s) to perform certain aspects of the functions described in the present disclosure.

When implemented in software, the techniques may be stored on a computer-readable medium as one or more instructions or codes, or may be transmitted through a computer-readable medium. The computer-readable media include both the computer storage media and the communication media including any medium that facilitates the transmission of a computer program from one place to another. The storage media may also be any available media that may be accessible to a computer. By way of non-limiting example, such a computer-readable medium may include RAM, ROM, EEPROM, CD-ROM or other optical disk storage, magnetic disk storage or other magnetic storage devices, or any other media that can be used to transmit or store desired program code in the form of instructions or data structures and can be accessible to a computer. In addition, any connection is properly referred to as a computer-readable medium.

For example, if the software is sent from a website, server, or other remote sources using coaxial cable, fiber optic cable, twisted pair, digital subscriber line (DSL), or wireless technologies such as infrared, wireless, and microwave, the coaxial cable, the fiber optic cable, the twisted pair, the digital subscriber line, or the wireless technologies such as infrared, wireless, and microwave are included within the definition of the medium. The disks and the discs used herein include CDs, laser disks, optical disks, digital versatile discs (DVDs), floppy disks, and Blu-ray disks, where disks usually magnetically reproduce data, while discs optically reproduce data using a laser. The combinations described above should also be included within the scope of the computer-readable media.

The software module may reside in RAM memory, flash memory, ROM memory, EPROM memory, EEPROM memory, registers, hard disk, removable disk, CD-ROM, or any other form of storage medium known. An exemplary storage medium may be connected to the processor such that the processor may read or write information from or to the storage medium. Alternatively, the storage medium may be integrated into the processor. The processor and the storage medium may exist in the ASIC. The ASIC may exist in the user terminal. Alternatively, the processor and storage medium may exist as separate components in the user terminal.

Although the examples described above have been described as utilizing aspects of the currently disclosed subject matter in one or more standalone computer systems, aspects are not limited thereto, and may be implemented in conjunction with any computing environment, such as a network or distributed computing environment. Furthermore, the aspects of the subject matter in the present disclosure may be implemented in multiple processing chips or apparatus, and storage may be similarly influenced across a plurality of apparatus. Such apparatus may include PCs, network servers, and portable apparatus.

Although the present disclosure has been described in connection with some examples herein, various modifications and changes can be made without departing from the scope of the present disclosure, which can be understood by those skilled in the art to which the present disclosure pertains. In addition, such modifications and changes should be considered within the scope of the claims appended herein.

## Claims

1. A method performed by one or more processors, the method comprising:
receiving medical image data, wherein the medical image data is associated with a plurality of body parts and configured for training a generative model for medical images;
acquiring label data associated with the medical image data, wherein the label data comprises a score associated with an anatomical location of at least one body part of the plurality of body parts; and
training, based on the received medical image data and the acquired label data, the generative model for medical images.

2. The method according to claim 1, wherein the medical image data comprises a plurality of two-dimensional (2D) computed tomography (CT) slice real images, and
wherein the generative model for medical images is trained to receive at least one of a number or a text representing a specific anatomical location of a body part and generate at least one 2D CT slice synthetic image associated with the specific anatomical location.

3. The method according to claim 2, further comprising:
generating, based on the generative model for medical images, the at least one 2D CT slice synthetic image associated with the specific anatomical location; and
outputting the generated at least one 2D CT slice synthetic image associated with the specific anatomical location.

4. The method according to claim 1, wherein the score comprises a body part regression (BPR) score quantifying an anatomical location of a body part associated with each of the medical image data.

5. The method according to claim 4, wherein the label data is generated by inputting the medical image data to a machine learning BPR model that is trained to estimate the BPR score.

6. The method according to claim 5, wherein the medical image data comprises a plurality of two-dimensional (2D) computed tomography (CT) slice real images and relative location information for each of the plurality of 2D CT slice real images, and
based on a determination that the machine learning BPR model fails to estimate a BPR score of a specific 2D CT slice real image of the plurality of 2D CT slice real images, a 2D CT slice real image of an adjacent location is identified using the relative location information, and the BPR score of the specific 2D CT slice real image is estimated based on the BPR score of the identified 2D CT slice real image of the adjacent location.

7. The method according to claim 1, further comprising normalizing the label data to a specific range.

8. The method according to claim 1, further comprising:
sampling, based on a specific body part regression (BPR) score or a specific range of BPR scores, the label data and the medical image data,
wherein the training the generative model for medical images comprises:
training, based on the sampled label data and the sampled medical image data, the generative model for medical images.

9. A non-transitory computer-readable recording medium storing instructions that, when executed, cause performance of the method according to claim 1.

10. A system for generating medical images associated with a plurality of body parts:
a memory; and
one or more processors connected to the memory and configured to execute one or more computer-readable programs included in the memory,
wherein the one or more programs include instructions for:
receiving a user input indicating a specific anatomical location of a body part of a plurality of body parts;
inputting the user input into a generative model for medical images to generate a medical image associated with the specific anatomical location; and
generating, based on the generative model for medical images, the medical image associated with the specific anatomical location,
wherein the generative model for medical images is a model that is trained based on medical image data and label data associated with the medical image data, and
wherein the label data comprises a score associated with an anatomical location of at least one body part of the plurality of body parts.

11. The system according to claim 10, wherein the label data and the medical image data are sampled based on a specific body part regression (BPR) score or a specific range of BPR scores, and
wherein the generative model for medical images is trained based on the sampled label data and the sampled medical image data.

12. The system according to claim 10, wherein the user input comprises at least one of a body part regression (BPR) score quantifying the specific anatomical location or a text indicating the specific anatomical location.

13. The system according to claim 12, wherein the user input comprises a single BPR score, and
wherein the generated medical image is a two-dimensional (2D) medical image corresponding to the specific anatomical location.

14. The system according to claim 12, wherein the user input comprises a specific range of BPR scores, and
wherein the generated medical image is a three-dimensional (3D) medical image corresponding to the specific anatomical location.

15. The system according to claim 10, wherein the generative model for medical images is a single generative model that is trained to generate medical images associated with the plurality of body parts.
